# EUROPEAN PATENT APPLICATION

(11) **EP 1 374 922 A1**
(43) Date of publication of application: **02.01.2004**
(21) Application number: 03254096.5
(22) Date of filing: 27.06.2003
(51) Int. Cl.: A61L 27/42, A61L 27/56, A61L 27/58, A61F 2/28

(54) **Polymer-bioceramic composite for orthopaedic applications and method of manufacture thereof**

(30) Priority: 29.06.2002 US 392488 P
(71) Applicant: Depuy Products, Inc., Warsaw, Indiana 46581 (US)
(72) Inventor: King, Richard, Warsaw, IN 46580 (US); Smith, Todd, Ft. Wayne, IN 46804 (US)
(74) Representative: Belcher, Simon James

(57) **Abstract**

A bioresorbable implantable bone repair structure comprises a porous ceramic matrix having a plurality of pores, and a polymer moulded into the plurality of pores of the porous ceramic matrix. The structure can be made by processes involving compression moulding to induce orientation of the polymer is multiple directions.

## Description

This invention pertains to polymer-bioceramic structures for use in the repair of bone. The structures are load bearing and may also be useful as drug delivery vehicles to facilitate the repair of bone defects. Processes for preparing the polymer-bioceramic structures by compression moulding are described.

The repair of bone defects is often accelerated by placing prosthetic implants in the defect site. If the prostheses are capable bearing loads associated with normal activity, such prostheses may alleviate the problems caused by prolonged, non-weight-bearing immobilization following injury, as well as decrease costs associated with extended hospitalization. Both naturally-occurring and artificially-produced prosthetic implants have been used to repair such defects. Naturally-occurring materials include grafts made from bones. The bone may be harvested directly from the patient, as in autograft-based procedures, or it may be harvested from a suitable donor, surrogate, or cadaver, as in allograft-based procedures. Natural bone is an ideal source of graft material not only for its biocompatibility, but also because natural bone grafts facilitate re-ossification of the defect site by promoting or conducting ingrowth of the patient's own bone tissue into the defect site. However, autograft bone implant procedures may be unavailable to certain patients who would be placed at increased risk by such procedures, typically requiring two surgical operations. Moreover, many of these patients, especially osteoporotic patients, are already compromised, and may not have a sufficient source of good quality bone that may be used for graft material.

Research has been directed toward the development of synthetic sources of material for use in bone defect repair. The design of a synthetic material that is chemically and morphologically similar to natural bone and exhibits similar mechanical properties is thought to provide the best source of graft material to repair most defects. The empirical composition of the mineral component of natural bone is:

Ca_{8.3}(PO₄)_{4.3}(HPO₄, CO₃)_{1.7}(OH, CO₃)_{0.3}.

This composition differs from the ideal stoichiometry for crystalline hydroxy apatite, as follows:

Ca₁₀(PO₄)₆(OH)₂.

The primary difference in the two chemical formulae is the presence of divalent ions such as (CO₃)²⁻ and (HPO₄)²⁻ that are found in natural bone, and replace trivalent (PO₄)³⁻ in crystalline hydroxy apatite. In addition, to the differences in the chemical formulas, natural bone is further characterized by the nanometre-sized crystalline morphology of the apatitic calcium phosphates present therein.

Chemical and morphological similarly between natural bone and the implant material tends to promote a strong implant/bone interface, such as high interface sheer strength. As the implant material diverges from these similarities, the implant/bone interface may be weaker. The weaker interface may arise from the patient's natural response to the implantation of xenographic material. The body of the patient will tend to isolate the implant if it is viewed as foreign material, often by reabsorption of the surrounding tissue and the subsequent formation of a fibrous tissue membrane at the interface between the implant and the natural bone. Such fibrous tissue formation at the interface interferes with the development of a strong mechanical interlock between the implant and the bone material surrounding the defect site. A better interface may be achieved when the implant material either allows or even promotes bone ingrowth into the defect site, providing a superior mechanical lock with the prosthesis. Ingrowth of the patient's bone into the implant may be facilitated by coating the implant with "bone-like" material, as disclosed in US-6136029, or by fabricating the entire implant from "bone-like" material. In the latter example, the implant is not only capable of superior interlock at the interface, but may also be completely replaced by the patients natural bone with time.

The formulae described above have been the basis for various synthetic bone substitutes, including poorly crystalline hydroxyapatite (PCA), as disclosed in US-6331312, and tricalcium phosphate (TCP). PCA and TCP have been reported to provide implants with bioactive surfaces that promote ingrowth of natural bone when implanted into bone. In addition, it has been observed that both PCA and TCP are reabsorbed by the host tissue.

In addition to bioceramic materials, organic polymers have been used as bone defect repair materials, including poly(methyl methacrylate) (PMMA), poly(lactic acid) (PLA), and poly(glycolic acid) PGA. PMMA, also commonly used as a bone cement, is not subject to degradation by most biological processes in the patient. However, PMMA-based compositions have been made partially resorbable by including cross-linked poly(propylene glycol fumarate) (PPF) and a particulate bioceramic, as disclosed in US-5085861 and US-4843112. However, these cements are primarily designed to be used in conjunction with the implantation of other non-resorbable prosthetic devices. Bone ingrowth into the cement helps to achieve better mechanical lock of those prostheses, though such ingrowth is typically limited to the exposed surface of the cement. In other variations, composite bone cements incorporating a bioresorbable particulate compound with a non-biodegradable polymeric resin are disclosed in US-4373217.

The present invention encompasses bioresorbable and implantable structures for use in the repair of bone defects. The structures comprise a porous bioceramic matrix and a polymer disposed therein. The polymer is disposed in the void volume created by the porous nature of the matrix, and is illustratively oriented in the pores. The bioceramic is illustratively an inorganic salt that includes the ions of calcium and phosphate, and in other aspects the bioceramic includes sulfate and carbonate. The polymer is illustratively a synthetic polymer, or a naturally occurring polymer, including polypeptides. Certain structures described herein are capable of being substantially or even completely reabsorbed by the patient via endogenous biochemical, biological, and metabolic processes, leading to a prosthesis that does not require subsequent removal following treatment of the bone defect.

In some cases, the pores are interconnecting, illustratively to a substantial degree, and may even form an open-cell configuration. The pores open onto surfaces of the matrix, and may be arranged in a predetermined pattern. The predetermined pattern is illustratively a pattern corresponding to a bone healing or bone remodelling, arranged along radii in various cross sections, or similar patterns. The pores may be macropores or micropores, and have diameters in the range of about 1 to 100 µm, or about 100 to 1000 µm. In certain embodiments, the void volume is uniformly distributed throughout the volume of the bioceramic matrix, and may comprise from about 30% to about 80%, or about 50% to about 70% of the volume. These structures are illustratively capable of bearing a load comparable to the tissue surrounding the defect, such as similarly-situated bone tissue of a similar configuration, or more particularly cortical bone. The disclosed structures may facilitate bone tissue ingrowth into the defect site, and subsequently, the gradual replacement of the implant material by native bone tissue.

The structures described herein may be fabricated by compression moulding the polymer into the matrix, illustratively by squeeze-flow moulding, or compression moulding in manner to induce orientation of the polymer in multiple directions within the structure, or by polymerization *in situ.* The bioceramic matrix is sufficiently rigid to be used in compression moulding processes. In addition, the structure described herein illustratively have high toughness, high creep resistance, and high flexibility.

The structures are also useful for delivering drugs to defect sites, including agents that facilitate or enhance the growth of bone, such as osteogenic agents, proteins involved in bone growth, and populations of cells. Substances capable of enhancing the effectiveness of the drugs may also be included. The structures may be used in a variety of defects, such as bone voids, fractures, maxillofacial defects, periodontal defects, and defects related to or arising from the removal of a bone or bone tumours.

The present disclosure encompasses bioresorbable and implantable structures for use in the repair of bone defects. The structures described herein comprise a porous bioceramic matrix and a polymer. The polymer is disposed in the porous bioceramic matrix.

Bioceramics useful in the invention are substantially non-toxic, biodegradable, bioerodable, and bioresorbable. The terms "biodegradable" and "bioerodable" as used herein similarly refer to a material property where biological, biochemical, metabolic processes, and the like may effect the erosion or degradation of the material over time. Such degradation or erosion is due, at least in part, to contact with substances found in the surrounding tissues, body fluids, and cells, or via cellular action, enzymatic action, hydrolytic processes, and other similar mechanisms in the body. The term "bioresorbable" as used herein refers to materials that are used by, resorbed into, or are otherwise eliminated from the body of the patient via existing biochemical pathways and biological processes. For example, in embodiments where the bioceramic comprises calcium phosphate, bioresorbed calcium phosphate may be redeposited as bone mineral, be otherwise reused within the body, or be excreted. It is understood that some materials become bioresorbable following biodegradation or bioerosion of their original state, as described above.

The term "biocompatible" as used herein refers to material that does not elicit a substantial detrimental response in the host, including but not limited to an immune reaction, such as an inflammatory response, tissue necrosis, and the like that will have a negative effect on the patient.

The salts used to prepare the bioceramics and the bioceramic matrices, fabricated therefrom are commercially available or are readily prepared via known procedures. Bioceramics include calcium salts of carbonate, sulfate, phosphate, and the like. Exemplary bioresorbable calcium salts effective in the composition of this invention include calcium carbonate, calcium sulfate, calcium sulfate hemihydrate, also known as plaster of Paris, and certain porous or precipitated forms of calcium phosphate, and the like. The porous bioceramic matrix may also be fabricated from any number of natural bone sources, such as autograft or allograft material, or synthetic materials that are compositionally related to natural bone.

Calcium phosphate ceramics are in general prepared by sintering more soluble calcium salts, for example Ca(OH)₂, CaCO₃, and CaHPO₄, with a phosphorus-containing compound such as P₂O₅. Such preparations of calcium phosphate ceramics are generally disclosed in US-3787900, US-4195366, US-4322398, US-4373217 and US-4330514.

Suitable calcium phosphates include, but are not limited to, calcium metaphosphate, dicalcium phosphate dihydrate, calcium hydrogen phosphate, tetracalcium phosphates, heptacalcium decaphosphate, tricalcium phosphates, calcium pyrophosphate dihydrate, crystalline hydroxy apatite, poorly crystalline apatitic calcium phosphate, calcium pyrophosphate, monetite, octacalcium phosphate, and amorphous calcium phosphate.

These chemical formulae also come in a range of crystalline morphologies, all of which may be used in fabricating the bioceramic matrix, as disclosed in US-6331312 and US-6027742. Such calcium phosphates have been described as poorly-crystalline calcium phosphate (PCA) with an apatitic structure. Other examples include tricalcium phosphate, tetracalcium phosphate and other mixed-phase or polycrystalline calcium phosphate materials referred to in US-4880610 and US-5053312. It is contemplated that bioactive glass compositions may also be used in combination with the above-described bioceramics and include SiO₂, Na₂O, CaO, P₂O₅, Al₂O₃, and CaF₂. It is appreciated that the above-described calcium salts may be used alone or may be mixed to prepare the bioceramics described herein

Bioceramics with particular chemical compositions that may form the structures described herein include calcium phosphate apatites, such as hydroxyapatite (HA, Ca₁₀(PO₄)₆(OH)₂) described by R. E. Luedemann et al., Second World Congress on Biomaterials (SWCB), Washington DC, 1984, p. 224, fluoroapatites, tricalciumphosphates (TCP), such as that sold under the trade mark Synthograft, dicalciumphosphates (DCP), and mixtures of HA and TCP, as described by E. Gruendel et al., ECB, Bologna, Italy, 1986, Abstracts, p. 5, p. 32); mixed-metal salts such as magnesium calcium phosphates, and beta-TCMP, as described by A. Ruggeri et al., Europ. Congr. on Biomaterials (ECB), Bologna, Italy, 1986, Abstracts, p. 86; aluminum oxide ceramics; bioglasses such as SiO₂-CaO-Na₂O-P₂O₅, e.g. Bioglass 45S (SiO₂ 45 wt %. CaO 24.5%, Na₂O 24.5% and P₂O₅ 6%) described by C. S. Kucheria et al., SWBC, Washington, D.C., 1984, p. 214, and glass ceramics with apatites (MgO 4.6 wt %, CaO 44.9%, SiO₂ 34.2%, P₂O₅ 16.3% and CaF 0.5%) described by T. Kokubo et al., SWBC, Washington, D.C., 1984, p. 351; bioceramics incorporating organic ions, such as citrate, as disclosed in US-5149368; and commercial materials, such as those sold under the trade marks Durapatite, Calcitite, Alveograf, and Permagraft.

Bioresorption of the foregoing may be facilitated by fabricating porous or channelled structures from these bioceramics. It is understood that the nature and size of these pores or channels may affect bioresorption. In certain aspects, the pores of the structure are interconnected forming an open-cell porous structure. It is understood that each of the foregoing materials possess differing bioresorption characteristics obtainable in the treatment subject and such characteristics may be advantageously chosen via routine experimentation for particular variations of the processes and methods described herein. It is also understood that both chemical composition and crystal morphology may affect bioresorption rates. For example, bioceramics fabricated from mixtures of calcium phosphate and calcium carbonate or calcium phosphate and calcium sulfate typically undergo resorption at higher rates than bioceramics fabricated from calcium phosphate alone. Furthermore, highly crystalline bioceramics typically undergo resorption at rates slower than poorly crystalline or amorphous bioceramics.

The porous microstructure of the bioceramics may be achieved by heat consolidation or sintering of bioceramic powders in appropriate moulds. The porous matrices may be macroporous or microporous. Microporous matrices typically have pores in the range from about 1 to about 100 µm in size, while macroporous matrices typically have pores in the range from about 100 to about 1000 µm in size. In certain embodiments the pore size in a given range is substantially uniform. The pores in the matrix account for the void volume thereof. Such void volume may be from about 30% to about 80%, and illustratively about 50% to about 70% of the matrix volume. The pores are typically interconnecting, and in some cases to a substantial degree. The pores may form an open-cell configuration in some embodiments. In embodiments where the void volume constitutes a substantial portion of the matrix volume, the pores are typically close together. Illustratively, adjacent pores are separated by less than 100 µm, and in other embodiments separated by less than about the average of the diameters of the adjacent pores.

The pores may be arranged in predetermined patterns that correspond to bone-healing or bone-remodelling patterns, Haversian systems, and other naturally-occurring patterns in bone. In some embodiments, the pores are aligned along radii in various cross-sections of the structures described herein.

In some cases, porous bioceramic matrices are commercially available, such as (1) those sold under the trade marks Pro Osteon 200 and Pro Osteon 500 (hydroxyapatite bone-graft substitutes having interconnected porous structures with pore sizes of 200 or 500 µm, similar to that of cancellous bone) available from Interpore International, Irvine, California; (2) Vitoss Blocks (calcium phosphate porous structure having ca. 90% porosity, with pore sizes from 1 to 1000 µm in diameter) available from Orthovita Inc., Malvern, Pennsylvania; and (3) synthetic porous hydroxyapatite (made by a foam process having controlled porosity and pore sizes) available from Hi-Por Ceramics, United Kingdom.

Polymers useful in the invention are preferably non-toxic, biocompatible, biodegradable, and bioresorbable, i.e., their degradation products are used by or are otherwise eliminated from the body of the treated subject via existing biochemical pathways and biological processes. The monomers used to prepare the polymers, and in some cases the polymers themselves, that are employed in the structures described herein are available commercially or are readily prepared through known procedures. Such polymers may be synthetic or naturally occurring, or may be polymer blends or copolymers.

Thermoplastic polymers useful herein include pharmaceutically-compatible polymers that are biodegradable, bioresorbable, and soften when exposed to heat but return to the original state when cooled. Examples include polylactides, polyglycolides, polycaprolactones, polyanhydrides, polyamides, polyurethanes, polyesteramides, polyorthoesters, polydioxanones, polyacetals, polyketals, polycarbonates, polyorthoesters, polyphosphazenes, polyhydroxybutyrates, polyhydroxyvalerates, polyalkylene oxalates, polyalkylene succinates, poly(malic acid), poly(amino acids), poly(methyl vinyl ether), poly(maleic anhydride), and copolymers, terpolymers, or combinations or mixtures therein.

Certain polyesters, polyanhydrides, polyorthoesters, and the like may be used in the structures described herein, for example as disclosed in US-3997512 (biodegradable polyester resin prepared by esterifying diglycolic acid with an unhindered glycol, providing self-supporting film forming properties for drug delivery), US-4181983 (biodegradable, assimilable, hydrophilic bandage for a dry socket in dental therapy), US-4481353 (bioresorbable polyesters composed of the Krebs Cycle components, such as succinic acid, fumaric acid, oxaloacetic acid, L-malic acid, and D-malic acid, and a diol, such as glycolic acid, L-lactic acid, and D-lactic acid), and US-4452973 (poly(glycolic acid)/poly(oxyalkylene) ABA triblock copolymers).

In particular, polyesters of alpha-hydroxycarboxylic acids, such as poly(L-lactic acid) (PLLA), poly(D,L-lactic acid) (PDLLA), polyglycolide (PGA), poly(lactide-glycolide) (PLGA), poly (D,L-lactide-trimethylene carbonate), and polyhydroxybutyrate (PHB), and polyanhydrides, such as poly(anhydride-imide), and copolymers thereof may be used to form the structures described herein. Generally, other available alpha hydroxy carboxylic acids can be used for making satisfactory polymers, including comonomers. Polylactic acid polymers may be used in polymer blends and include other materials present in a minor proportion such as glycolide, beta-propiolactone, tetramethylglycolide, beta-butyrolactone, gamma-butyrolactone, pivalolactone, and intermolecular cyclic esters of alpha hydroxybutyric acid, alpha hydroxyisobutyric acid, alpha hydroxyvaleric acid, alpha hydroxyisovaleric acid, alpha hydroxycaproic acid, alpha hydroxy-alpha-ethylbutyric acid, alpha hydroxyisocaproic acid, alpha hydroxy-beta-methylvaleric acid, alpha hydroxyheptanoic acid, alpha hydroxyoctanoic acid, alpha hydroxydecanoic acid, alpha hydroxymyristic acid, alpha hydroxystearic acid, alpha hydroxylignoceric acid, and beta-phenyllactic acid. It is appreciated that each of the foregoing synthetic polymers may be used as a component monomer in the preparation of a copolymer of other synthetic polymers or of the naturally-occurring polymers described below.

Suitable bioerodable polymers of natural origin for use as a matrix in the composite include, but are not limited to, collagen, glycogen, chitin, chitosan, celluloses, starch, keratins, silk, alginate, polypeptides, such as poly(arginine), poly(glutamic acid), poly(glutamine), poly(lysine), and certain polycarbonate copolymers of tyrosine and other tyrosine-containing peptides, and polynucleotides. It is appreciated that each of the foregoing naturally-occurring polymers may be used as a component monomer in the preparation of a copolymer of other naturally-occurring polymers or of the synthetic polymers described above.

Hydrogels such as the poloxamers and pluronics may be used alone or in combination with the above polymers in fabricating the structures described herein. Furthermore, additional materials may be added to the polymer component including commercially available products such as those sold under the trade marks Endobone (Merck), Hapset (Lifecore Biomedical), SRS (Norian), Bonesource (Leibinger), Collograft (Zimmer), and Osteograf (CereMed), or demineralized bone matrix, derivatized hyaluronic acid.

It is contemplated that the rate of biodegradation, bioerosion, or bioresorption of the polymer component of the structures described herein, or the rate of release of bioactive agents incorporated in the structures described herein may be controlled by varying either the type of or molecular weight of the polymer or copolymer components, by including a release rate modification agent, or by varying the combination and concentrations of ingredients that comprise the polymer itself. For example, poly(lactic acid)-based polymers typically undergo resorption at rates slower than poly(glycolic acid)-based polymers. Resorption rates may be manipulated by choice of the ratio of the mixture of such polymer components. Furthermore, the polymer may be disposed in the structure in a radially varying manner, such that resorption characteristics vary from the centre of the structure to the perimeter of the structure. In certain embodiments, it is appreciated that a perimeter that resorbs rapidly and allows rapid infiltration of bone ingrowth at the perimeter relative to the interior may be desirable. Such configurations may be fabricated as described herein by varying the polymer component, such as by varying the polymer molecular weight distribution or polymer, copolymer, or polymer blend composition as a function of cross-section of the structure.

In addition, it is understood that polymers, copolymers, and polymer blends used in the structures described herein may be selected for particular drug release characteristics, mechanical reinforcement capabilities, or mechanical properties such as elasticity, or combinations thereof depending on the desired configuration of the structure. Still other bioresorbable organic polymers are disclosed in US-5385887, US-4578384, US-4563489, US-4637931, US-4578384 and US-5084051.

Structures described herein are desirably chemically biocompatible; capable of supporting a load; accept or facilitate bone ingrowth promoting good mechanical interlock; and capable of complete or near complete resorption by the patient and contemporaneous replacement by natural bone in the patient.

Biocompatible calcium phosphate ceramics are selected particularly in bone repair embodiments for their properties to promote interfacial osteoconduction. Bone ingrowth is facilitated by an embodiment where the bioceramic matrix is a three-dimensional scaffold possessing pores, interstices, pockets, channels, passages, tunnels, and the like. In some aspects, these interstices, pockets, channels, passages, tunnels, and the like comprise a major portion, or a substantial portion of the volume possessed by the porous bioceramic matrix. In other aspects, these interstices, pockets, channels, passages, tunnels, and the like comprise less than 50% of the volume possessed by the porous bioceramic matrix.

In one embodiment, the polymer is disposed in the porous bioceramic matrix in a manner to substantially fill the volume of available interstices in the matrix. The polymer disposed in the porous bioceramic matrix may provide reinforcement of the load-bearing capability of the structures described herein. In addition, in embodiments where the porous bioceramic matrix is a substantially rigid scaffold, the polymer may provide a degree of elasticity to decrease the brittleness of the bioceramic structures described herein that is not imparted to the structures from the porous bioceramic matrix component. In one aspect, the polymer component and the bioceramic component are substantially regularly distributed throughout the cross-section of the structures described herein. In another aspect, the polymer component and the bioceramic component are substantially distributed according to an organized pattern throughout the cross-section of the structures described herein. However, it is appreciated that the distribution of the two components may be irregular or regularly varying relative to a pattern in variations of the structures described herein. The polymer disposed in the matrix may possess molecular orientation along an axis, or may be oriented in multiple directions in a plane or in space.

The composite structures described herein are load bearing. These structures may bear loads similar in magnitude to that able to be borne by the tissue surrounding the defect, such as a bone structure of similar dimensions, or a bone structure consisting primarily of cortical bone. The structures described herein may also possess mechanical properties similar to that of natural bone, or in particular cortical bone. These mechanical properties include, but are not limited to, tensile strength, impact resistance, Young's modulus, compression strength, sheer strength, stiffness, and the like. It is appreciated that structures described herein possessing mechanical properties similar to those exhibited by the tissue surrounding such implanted structures may favourably influence the stress-shielding effect.

It is understood that additional materials may be added to the polymer matrix in order to increase its load-bearing capacity or capability, such as carbon fibres or other reinforcing fibres.

In yet another embodiment, the composite contains a radiographic supplemental material for imaging the implant in vivo. Such supplementary material may be included in the polymer component or the porous bioceramic matrix component or both. Suitable electron dense materials include materials known in the art, such as titanium oxide, barium sulfate, zirconium oxide, and the like in clinically relevant concentrations.

While it is appreciated that the above-described composition may also elicit osteogenic behaviour on its own, another embodiment is a structure that may be used as a drug delivery system. Either the polymer, the bioceramic, or both may include a biologically-active agent, either singly or in combination, such that the composite structure or implant will provide a delivery system for the agent. The agent may be delivered to adjacent tissues or tissues proximal to the implant site. Biologically-active agents which may be used alone or in combination in the implant precursor and implant include, for example, a medicament, drug, or other suitable biologically-, physiologically-, or pharmaceutically-active substance which is capable of providing local or systemic biological, physiological, or therapeutic effect in the body of the patient. The biologically-active agent is capable of being released from the solid implant matrix into adjacent or surrounding tissue fluids during biodegradation, bioerosion, or bioresorbtion as described above.

In one aspect, the biologically-active agent is an osteogenic agent. Each component substance, the bioceramic matrix material or the polymer, may be osteogenic; or the combination of the bioceramic matrix material with the polymer forming the structure described above may be osteogenic.

The term "osteogenic agent" as used herein refers to agents that promote, induce, stimulate, generate, or otherwise effect the production of bone or the repair of bone. The presence of an osteogenic agent in the defect site may elicit an effect on the repair of the defect in terms of shortening the time required to repair the bone, by improving the overall quality of the repair, where such a repair is improved over situations in which such osteogenic agents are omitted, or may achieve contemporaneously both shortened repair times and improved bone quality. It is appreciated that osteogenic agents may effect bone production or repair by exploiting endogenous systems, such as by the inhibition of bone resorption.

Osteogenic agents may promote bone growth by acting as bone anabolic agents. Compositions of the present invention may also effect repair of the bone defect by stabilizing the defect to promote healing. The ramifications of using such osteogenic agents include increased healing rates, effecting a more rapid new bone ingrowth, improved repair quality, or improved overall quality of the resulting bone.

In one embodiment the osteogenic agent is a "small molecule" such as a synthetic molecule, drug, or pharmaceutical involved in, or important to, bone biology, including statins, such as lovastatin, simvastatin, atorvastatin, and the like, fluprostenol, vitamin D, estrogen, a selective estrogen receptor modifier, or a prostaglandin, such as PGE-2. Combinations of such small molecules in providing the osteogenic agent are contemplated herein.

In another embodiment the osteogenic agent is a "large molecule" such as an endogenous-derived protein or other protein, an enzyme, a peptide, receptor ligand, a peptide hormone, lipid, or carbohydrate involved in, or important to, bone physiology, including the bone morphogenic or bone morphogenetic proteins (BMPs), such as BMP-2, BMP-7, and BMP-9, chrysalin, osteogenic growth peptide (OGP), bone cell stimulating factor (BCSF), KRX-167, NAP-52, gastric decapeptide, parathyroid hormone (PTH), a fragment of parathyroid hormone, osteopontin, osteocalcin, a fibroblast growth factor (FGF), such as basic fibroblast growth factor (bFGF) and FGF-1, osteoprotegerin ligand (OPGL), platelet-derived growth factor (PDGF), an insulin-like growth factor (IGF), such as IGF-1 and IGF-2, vascular endothelial growth factor (VEGF), transforming growth factor (TGF), such as TGF-alpha and TGF-beta, epidermal growth factor (EGF), growth and differentiation factor (GDF), such as GDF-5, GDF-6, and GDF-7, thyroid-derived chondrocyte stimulation factor (TDCSF), vitronectin, laminin, amelogenin, amelin, fragments of enamel, or dentin extracts, bone sialoprotein, and analogs and derivatives thereof. Combinations of such large molecules in providing the osteogenic agent are contemplated herein.

In another embodiment the osteogenic agent is a cell or population of cells involved in, or important to, bone biology, such as pluripotent stem cells, autologous, allogenic, or xenogeneic progenitor cells, chondrocytes, adipose-derived stem cells, bone marrow cells, mesenchymal stem cells, homogenized or comminuted tissue transplants, genetically transformed cells, and the like. Bone powders, including demineralized bone powders and bone matrix, may also be used. Combinations of such cell populations in providing the osteogenic agent are also contemplated herein.

Depending upon its nature, the osteogenic agent may be present in the structure within the range from about 0.1% to about 30% by weight, preferably in the range from about 1% to 9% by weight.

Any of a variety of medically or surgically useful substances can also be incorporated into the osteogenic components described herein. It is contemplated that such additives may serve to reduce barriers to repair and thus maximize the potential of the osteogenic agent.

Components that are capable of preventing infection in the host, either systemically or locally at the defect site, are contemplated as illustrative useful additives. These additives include anti-inflammatory agents, such as hydrocortisone, prednisone, and the like, NSAIDS, such as acetaminophen, salicylic acid, ibuprofen, and the like, selective COX-2 enzyme inhibitors, antibacterial agents, such as penicillin, erythromycin, polymyxin B, viomycin, chloromycetin, streptomycins, cefazolin, ampicillin, azactam, tobramycin, cephalosporins, bacitracin, tetracycline, doxycycline, gentamycin, quinolines, neomycin, clindamycin, kanamycin, metronidazole, and the like, antiparasitic agents such as quinacrine, chloroquine, vidarabine, and the like, antifungal agents such as nystatin, and the like, antiviricides, particularly those effective against HIV and hepatitis, and antiviral agents such as acyclovir, ribarivin, interferons, and the like.

Systemic analgesic agents such as salicylic acid, acetaminophen, ibuprofen, naproxen, piroxicam, flurbiprofen, morphine, and the like, and local anaesthetics such as cocaine, lidocaine, bupivacaine, xylocaine, benzocaine, and the like, are also contemplated as additives.

Other additional ingredients that may enhance the overall effectiveness of the osteogenic agent include amino acids, peptides, including peptide fragments of the various bone morphogenetic proteins, vitamins, inorganic elements, co-factors for protein synthesis, hormones, enzymes such as collagenase, peptidases, oxidases, and the like, angiogenic drugs and polymeric carriers containing such drugs, biocompatible surface active agents; anti-thrombotic drugs, cytoskeletal agents, natural extracts, bioadhesives, anti-tumour agents, antineoplastic agents, such as methotrexate, 5-fluorouracil, adriamycin, vinblastine, cisplatin, and the like, tumour-specific antibodies conjugated to toxins, tumour necrosis factor, cellular attractants and attachment agents; immuno-suppressants, permeation and penetration enhancers, such as fatty acid polyethylene glycol monoesters of laureate, myristate, stearate, and the like, and nucleic acids. The amounts of such added substances can vary widely with optimum levels being readily determined for a given case by routine experimentation.

Still other additional ingredients, or metabolic precursors thereof, that are capable of promoting growth and survival of cells and tissues, or augmenting the functioning of cells, are contemplated, and include nerve growth promoting substances, such as a ganglioside, nerve growth factor, and the like, fibronectin (FN), growth hormones, such as somatotropin, human growth hormone (HGH), and the like, colony stimulating factors, cytokines, and interleukin-1 (IL-1).

It is appreciated that the release rate of the drug from a delivery system based on the present structures may also be a function of the degree of cross-linking present in the polymer, the nature and concentration of the drug substance in the polymer, particulate size/solubility, nature/biodegradability of polymer component, and the "in vivo environment" of the implanted structure.

After implantation, the structure is subject to biodegradation, bioerosion, and contemporaneous bioresorption. Thus, as voids are formed in the structure, bone ingrowth will tend to fill these voids to generate a desirable mechanical interlock. In addition, as the bone ingrowth advances, more of the structure is exposed to the bioresorption processes present in the patient. At some time point, the extent of the new bone ingrowth reaches a level where all of or substantially all of the implant structure has been replaced by natural bone tissue.

The structures described herein are suitable for repairing bone voids, fractures, non-union fractures, periodontal defects, maxillofacial defects, arthrodesis, and the like. In addition, structures described herein may be used as reinforcement of bone fractures, dental implants, bone implants, bone prostheses and the like. It is appreciated that structures described herein may also be generally used in conjunction with other traditional fixation, immobilization, prosthetic methods.

Fractures that may be treated by the structures described herein include fractures of the proximal humerus, diaphyseal humerus, diaphyseal femur, trochanteric femur, and trochanteric humerus. In addition, the structures may be used in the repair of osteoporosis-induced fractures, including those that involve a crushing-type injury, such as vertebral fractures, and the like. In such fractures, the porous osteoporotic bone collapses into itself typically causing a void or bone defect at the site of the fracture. In order to achieve secure stabilization of the fracture, the bone defect may be filled with a structure described herein.

In addition, the structures can also be employed in the treatment of bone voids resulting from bone tumours. The tumour may leave a bone defect in the form of a void, or the tumour may be surgically removed, potentially with surrounding tissue, to leave a void or cavity. The cavity or void may be filled with a structure described herein to treat the defect.

The structures described herein may be fabricated by using known methods. In particular the structures may be fabricated by compression moulding and polymerization *in situ.* Compression moulding processes include transfer moulding and squeeze-flow moulding.

In one aspect, the structure is fabricated by compression moulding. A fully-consolidated biocompatible polymer, in a machined-block form, is placed on top of a bioceramic matrix in a mould cavity. The mould is then heated to a temperature at about or above the melting temperature of the polymer. Minimal loading occurs during the heating step. Pressurization of the mould is initiated once the molten polymer is fluid enough for diffusion through the porous structure. In addition, vacuum may be optionally applied during this process to prevent degradation or hydrolysis of biocompatible polymer. It is appreciated that applying a vacuum may also facilitate the diffusion of polymer into the matrix.

In another aspect, the structure is fabricated by transfer moulding. A fully-consolidated biocompatible polymer, in a machined-block form, is preheated to a temperature at about or above the melting temperature of the polymer and subsequently transferred to a preheated mould cavity containing a porous bioceramic matrix. Once the molten polymer is positioned, squeeze moulding is initiated by applying a load to a plunger, thereby pressurizing the mould cavity.

In another aspect, the structure is fabricated by flow moulding. A porous bioceramic matrix having a small-diameter open core is used. In addition, the matrix has interconnected channels that are also connected to the open core. The channels are arranged in a substantially radial pattern when viewed in a given cross section of the matrix. The porous matrix is placed in a mould cavity and polymer is disposed into the open core by either of the above-described methods of compression moulding or transfer moulding. In either case this process allows orientation of the polymer from in the matrix. Such orientation may further reinforce and favourably influence the mechanical properties of the structures described herein.

The polymer may be disposed in the porous matrix by injecting polymer precursor or monomer into the matrix, then effecting in-situ polymerization of the polymer precursor or monomer. Such polymers which are derived *in situ* may also be optionally cross-linked. Excess solvent accompanying certain in situ polymerization processes may be removed using standard procedures, including removal by evaporation, freeze-drying/freeze-thawing cycles, and the like.

Once the polymer is disposed in the porous matrix by any of the methods described herein, including compression moulding, transfer moulding, squeeze-flow moulding, and in-situ polymerization, the polymer may be optionally cross-linked. Crosslinking may be accomplished by any of the variety of known methods, including treatment with heat or irradiation, such as X-ray radiation, gamma irradiation, electron beam radiation, and the like.

The structures described herein include the formation of bulk material that may be shaped by the medical practitioner on site, or various prefabricated shapes ready or near ready for implantation. Such bulk material may in the form of bars, blocks, billets, sheets, and the like. Such shapes include plates, plugs, cubes, cylinders, pins, tubes, chutes, rods, screws, including screws of the kind disclosed in US-6162225 (bone screw fabricated from allograft bone). In addition, shapes that tend to mimic the overall dimensions of the bone may be fabricated. Shapes that tend to mimic the overall dimensions of the bone are particularly useful in the repair of fractures at risk of non-union. Such bulk shapes or particularly-dimensioned shapes may be obtained by employing mould cavities possessing such dimensions. Alternatively, the particularly-dimensioned shapes may be fabricated by machining the bulk stock.

## Claims

1. A bioresorbable implantable bone repair structure, the structure comprising a porous ceramic matrix having a plurality of pores, and a polymer moulded into the plurality of pores of the porous ceramic matrix.

2. The structure of claim 1, wherein the polymer is compression moulded into the plurality of pores of the porous ceramic matrix.

3. The structure of claim 1, wherein the polymer is transfer moulded into the plurality of pores of the porous ceramic matrix.

4. The structure of claim 1, wherein the polymer is squeeze-flow compression moulded into the plurality of pores of the porous ceramic matrix.

5. The structure of claim 1, wherein the polymer is oriented in multiple directions in the plurality of pores of the porous ceramic matrix.

6. The structure of claim 1, wherein a number of the plurality of pores are interconnecting.

7. The structure of claim 1, wherein the plurality of pores form an open-cell configuration.

8. The structure of claim 1, wherein the plurality of pores of the pores open onto a substantial number of the exterior surfaces of the ceramic matrix.

9. The structure of claim 1, wherein the plurality of pores comprises macropores.

10. The structure of claim 1, wherein the plurality of pores comprises micropores.

11. The structure of claim 1, wherein each of the plurality of pores has a diameter in the range from about 1 to about 1000 µm.

12. The structure of claim 1, wherein each of the plurality of pores has a diameter in the range from about 100 to about 1000 µm.

13. The structure of claim 1, wherein the porous ceramic matrix comprises a bioresorbable substance selected from the group consisting of hydroxyapatite, betatricalcium phosphate, calcium sulfate, and calcium carbonate.

14. The structure of claim 1, wherein the polymer is a water soluble polymer or a hydrophilic polymer.

15. The structure of claim 1, wherein the polymer is selected from the group consisting of poly(ethylene oxide), poly(N-vinylpyrrolidinone), poly(vinylalcohol), poly-(lactic acid), poly(L-lactic acid), poly(glycolic acid), polycaprolactone, poly(hydroxycaproic acid), polydioxanone, bioresorbable polycarbonate, poly(trimethylene carbonate), polypeptides, (ethylene oxide-propylene oxide) block copolymers, and copolymers thereof, and collagen and gelatin.

16. The structure of claim 1, wherein the polymer is a polypeptide containing tyrosine, lysine, arginine, glutamine, glutamic acid, or a combination thereof.

17. The structure of claim 1, further comprising a drug.

18. The structure of claim 17, wherein the drug is incorporated in the polymer.

19. The structure of claim 17, wherein the drug is present at a higher concentration at the perimeter of the structure than in the interior of the structure.

20. The structure of claim 19, wherein the drug is present in the structure as a concentration gradient, the gradient increasing from the interior of the structure to the perimeter of the structure.

21. The structure of claim 17, wherein the drug comprises an osteogenic agent.

22. The structure of claim 17, wherein the drug is a protein, protein fragment, or peptide.

23. The structure of claim 22, wherein the protein is selected from the group consisting of bone morphogenetic proteins, parathyroid hormone, and growth factors.

24. The structure of claim 17, wherein the drug is a peptide fragment of a bone morphogenetic protein.

25. The structure of claim 17, wherein the drug comprises an osteogenic agent, and an additional ingredient, where said additional ingredient is capable of enhancing the efficacy of the drug.

26. The structure of claim 25, wherein the additional ingredient is selected from the group consisting of antibiotics, anti-inflammatory agents, and analgesics.

27. The structure of claim 1, further comprising a population of cells.

28. A process for fabricating a bioresorbable implantable bone repair structure, the process comprising the step of moulding a polymer into a plurality of pores of a porous ceramic matrix.

29. The process of claim 28, wherein the moulding step comprises compression moulding the polymer into the plurality of pores of the porous ceramic matrix.

30. The process of claim 28, wherein the moulding step comprises transfer moulding the polymer into the plurality of pores of the porous ceramic matrix.

31. The process of claim 28, wherein the moulding step comprises squeeze-flow compression moulding the polymer into the plurality of pores of the porous ceramic matrix.

32. The process of claim 28, wherein the moulding step comprises positioning a polymer block and the porous ceramic matrix in a mould, and compression moulding the polymer block and the porous ceramic matrix.

33. The process of claim 28, wherein the moulding step comprises compression moulding a polymer block in a molten state.

34. The process of claim 28, wherein the moulding step comprises inducing an orientation in the polymer within at least a portion of the plurality of pores of the porous ceramic matrix.

35. The process of claim 28, wherein the ceramic matrix comprises a bioresorbable substance selected from the group consisting of hydroxyapatite, betatricalcium phosphate, calcium sulfate, and calcium carbonate.

36. The process of claim 28, wherein the polymer is selected from the group consisting of poly(ethylene oxide), poly(N-vinylpyrrolidinone), poly(vinylalcohol), poly(lactic acid), poly(L-lactic acid), poly(glycolic acid), polycaprolactone, poly(hydroxycaproic acid), polydioxanone, bioresorbable polycarbonate, poly(trimethylene carbonate), polypeptides, (ethylene oxide-propylene oxide) block copolymers, and copolymers thereof, and collagen and gelatin.

37. The process of claim 28, wherein the polymer comprises a drug.

38. The process of claim 28, wherein the polymer comprises an osteogenic agent.

39. A process for fabricating a bioresorbable implantable bone repair structure, the process comprising the steps of:
disposing a polymer precursor into a plurality of pores of a porous ceramic matrix, and
polymerizing the polymer precursor in the plurality of pores of the porous ceramic matrix.
